# EUROPÄISCHE PATENTSCHRIFT

(11) **EP 3 023 705 B1**
(45) Veröffentlichungstag und Bekanntmachung des Hinweises auf die Patenterteilung: **10.08.2022**
(21) Anmeldenummer: 15156137.0
(22) Anmeldetag: 23.02.2015
(51) Int. Cl.: F24F 6/12, F24F 7/08, F24F 12/00, F24F 13/06

(54) **PRODUKTIONSSTÄTTE UMFASSEND EIN GESCHLOSSENES GEBÄUDE SOWIE EINE LÜFTUNGSANLAGE UND VERFAHREN ZUM KLIMATISIEREN EINES SOLCHEN GEBÄUDES**
PRODUCTION SITE COMPRISING A CLOSED BUILDING AND A VENTILATION SYSTEM AND METHOD FOR AIR-CONDITIONING SUCH A BUILDING
SITE DE PRODUCTION COMPRENANT UN BÂTIMENT FERMÉ ET UNE INSTALLATION DE VENTILATION ET PROCÉDÉ DE CLIMATISATION D'UN TEL BÂTIMENT

(30) Priorität: 18.11.2014 DE 202014105531 U
(43) Veröffentlichungstag der Anmeldung: 25.05.2016
(73) Patentinhaber: Reichenbach, Albert, 58675 Hemer (DE); Englhardt, Norbert, 80333 München (DE); Schmidt, Markus, 81667 München (DE)
(72) Erfinder: Reichenbach, Albert, 58640 Iserlohn (DE); Lösche, Klaus, 27580 Bremerhaven (DE); Englhardt, Norbert, 80333 München (DE); Schmidt, Markus, 81667 München (DE)
(74) Vertreter: Haverkamp Patentanwälte PartG mbB

(56) Entgegenhaltungen:
- WO-A1-99/06774

## Beschreibung

Die Erfindung betrifft eine Produktionsstätte umfassend ein geschlossenes Gebäude, in dem ein, vorzugsweise mehrere unterschiedliche Arbeitsbereiche angeordnet sind, sowie eine Lüftungsanlage zum Fördern einer Luftströmung in dem Gebäude. Die Erfindung betrifft des Weiteren ein Verfahren zum Klimatisieren eines geschlossenen Gebäudes einer Produktionsstätte.

Produktionsstätten befinden sich in aller Regel in geschlossenen Gebäuden, in denen die einzelnen Arbeitsbereiche oftmals in unterschiedlichen Produktionsebenen angeordnet sind. In vielen Fällen wird es erforderlich sein, die Arbeitsbereiche zu klimatisieren oder zumindest eine Frischluftzufuhr in den Arbeitsbereichen einzurichten. Bei mehrgeschossigen Gehäusen ist es üblich, jedem Geschoss eine Lüftungs- und/oder Klimaanlage zuzuordnen, auch um die einzelnen Produktionsebenen unabhängig voneinander belüften und/oder klimatisieren zu können. Bei Produktionsstätten ist es mitunter auch erforderlich, die relative Luftfeuchte in allen oder auch nur in manchen Arbeitsbereichen auf einem bestimmten Niveau zu halten. Dann wird der Lüftungsanlage eine Luftbefeuchtungsanlage zugeordnet sein. Die Luftfeuchte wird in Dampfform in das Gebäude bzw. den hinsichtlich der relativen Luftfeuchte zu überwachenden Arbeitsbereich eingebracht. Problematisch ist bei einer solchermaßen realisierten Luftbefeuchtung die Gefahr einer damit einhergehenden Kondensatbildung. Feuchtigkeitskondensatbildung ist vor allem bei solchen Produktionsstätten unerwünscht, in denen Lebensmittel hergestellt oder verarbeitet werden.

Für die Frischluftzufuhr in Gebäuden werden auch Lüftungsanlagen eingesetzt, bei denen über einen im Gebäude angeordneten Luftaustrittskanal ein Zuluftstrom in das Gebäude eingeleitet wird. Ferner umfasst eine solche Lüftungsanlage ein oder mehrere, hinsichtlich ihrer freien durchströmbaren Querschnittsfläche einstellbare Abluftöffnungen, durch die Abluft aus dem Gebäude austreten kann. Der wenigstens eine Luftaustrittskanal zum Zuführen der Zuluft ist mit Abstand zu der zumindest einen Abluftöffnung angeordnet, so dass sich bei einem Betrieb der Lüftungsanlage in dem Gebäude eine Luftströmung von dem zumindest einen Luftaustrittskanal zu der zumindest einen Abluftöffnung einstellt. Als Luftaustrittskanäle werden typischerweise poröse Kanäle, etwa Textilrohre eingesetzt.

Mit einer solchermaßen konzipierten Lüftungsanlage wird nicht nur Frischluft in den Innenraum zugeführt, sondern dieser wird zugleich durch die sich einstellende Luftströmung sauber gehalten. Staub oder andere Schwebstoffe werden mit dem Abluftstrom weggeführt. Aus diesem Grunde werden derartige Lüftungsanlagen vielfach auch in Lebensmittel herstellenden Betrieben eingesetzt, beispielsweise Bäckereien, insbesondere Großbäckereien. In Abhängigkeit von der mit einem solchen Abluftstrom abgeführten Schwebstoffe können ein oder mehrere Filter vorgesehen sein, durch die der Abluftstrom tritt, bevor er, falls nicht vollständig rezirkuliert, über die Abluftöffnung an die Umgebung abgegeben wird.

Die Lüftungsanlage kann über ein Wärmerückgewinnungsaggregat - einen so genannten Rekuperator - verfügen, der aus dem Abluftstrom Wärme entnimmt und an den Zuluftstrom abgibt. Auf diese Weise kann in dem Innenraum generierte Prozesswärme, etwa durch den Betrieb Wärme abgebender Geräte genutzt werden, um der Zuluft Wärme zuzuführen. Mitunter verfügt eine solche Lüftungsanlage zusätzlich auch über eine Heizeinrichtung zum autarken Erwärmen der zuzuführenden Zuluft.

Lüftungsanlagen dieser Art müssen mitunter durch eine Klimaanlage ergänzt werden, wenn die Temperaturen im Innenraum entweder durch entstehende Prozesswärme oder durch Umgebungswärme über eine tolerablen Temperaturschwelle ansteigen können.

WO 99/06774 A1 offenbart ein Vernebelungssystem als klimatisierende Kühleinrichtung. Dieses vorbekannte System umfasst ein Rohr oder einen Schlauch, aus dem darin eingebrachte Luft unter Druck herausgedrückt wird, sodass auf diese Weise ein Luftstrom generiert wird. Über die Länge eines solchen Luftaustrittsschlauches verteilt befinden sich mit Abstand zueinander eine Reihe von Dispersionsöffnungen. Diese sind als Düsen ausgelegt. Angeschlossen sind diese Düsen an eine Flüssigkeitszuführleitung, über die mittels einer Pumpe an den Düsen zu zerstäubende Flüssigkeit zugeführt wird. Aufgrund des Luftstromes, in dem diese Düsen angeordnet sind, wird die in den Düsen zerstäubte Flüssigkeit mit dem Luftstrom mitgeführt. Durch den Flüssigkeitsnebel, der in warmer Umgebung verdampft, wird der Umgebung Wärme entzogen, was für den mit diesem vorbekannten Verneblungssystem gewünschten Kühleffekt verantwortlich ist. Unterhalb der Düsen befindet sich eine Flüssigkeitsauffangwanne, um Flüssigkeitstropfen, die aus oder von den Düsen herabtropfen, aufzufangen.

Eine Einrichtung zum Aufrechterhalten eines bestimmten Feuchtigkeitsgehaltes in der Umgebungsluft ist aus FR 2 847 968 A1 bekannt. Bei diesem Gerät dient ein Ultraschallvernebler zum Erzeugen von Aerosoltröpfchen, die über einen Schlitz, der sich innerhalb des geförderten Luftstroms befindet austreten und von diesem mitgerissen werden. Eingesetzt wird dieses Gerät zum Aufrechterhalten der Luftfeuchtigkeit in einem Holzweinfässer enthaltenden Raum. Dieses vorbekannte Gerät ist als Umwälzgerät ausgelegt und führt keine Zuluft zu.

Auch wenn mit derartigen Lüftungs- bzw. Klimatisierungsanlagen auch Produktionsstätten ausgerüstet sind, wäre es wünschenswert, eine Lüftung bzw. Klimatisierung weniger aufwendig und weniger kostenträchtig gestalten zu können. Zudem wäre es wünschenswert, wenn die Betriebskosten reduziert werden könnten.

Ausgehend von diesem diskutierten Stand der Technik liegt der Erfindung daher die Aufgabe zugrunde, eine eingangs genannte, gattungsgemäße Produktionsstätte sowie ein eingangs genanntes, gattungsgemäßes Verfahren zum Klimatisieren einer solchen Produktionsstätte dergestalt weiterzubilden, dass ein Lüftungs- bzw. Klimatisierungsbetrieb insbesondere kostengünstiger durchgeführt werden kann.

Gelöst wird diese Aufgabe erfindungsgemäß durch eine Produktionsstätte mit den Merkmalen des Anspruchs 1 sowie durch ein Verfahren mit den Merkmalen des Anspruchs 8. Bevorzugte Ausführungsformen sind in den Unteransprüchen definiert.

Bei dieser Produktionsstätte und gleichermaßen bei dem beanspruchten Verfahren wird Luftfeuchte in Form eines Aerosols in den Innenraum eingebracht. Aus diesem Grunde umfasst diese Lüftungsanlage einen Nebelgenerator zum Erzeugen des Aerosols aus einer Flüssigkeit. Bei dem Nebelgenerator handelt es sich um einen Ultraschallvernebler. Ein solcher Ultraschallvernebler erzeugt mittels hochfrequenter mechanischer Schwingungen die Flüssigkeitströpfchen in der gewünschten Größenordnung aus einem Flüssigkeitsfilm. Eingesetzt werden können hierzu piezokeramische Elemente, die elektrische Schwingungen in die notwendigen mechanischen Schwingungen umwandeln. Von Bedeutung ist, dass das in dem Gebäude angeordnete Aerosolaustrittsrohr in Bezug auf das Luftaustrittsrohr, aus dem die Zuluft austritt, dergestalt angeordnet ist, dass das austretende Aerosol von der sich bei einem Betrieb der Lüftungsanlage in dem Gebäude einstellenden Luftströmung erfasst wird. Zusätzlich ist bei dieser Lüftungsanlage vorgesehen, dass die Tröpfchengröße des Aerosols eingestellt wird beziehungsweise eingestellt ist, dass diese von der sich in dem Innenraum des Gebäudes einstellenden Luftströmung mitgeführt werden können, mithin durch diesen in Schwebe gehalten werden. Dieses Zusammenwirken zwischen der Tröpfchengröße und der Anordnung des Aerosolaustritts in Bezug auf den Zuluftaustritt trägt Sorge dafür, dass eine unerwünschte Aerosolansammlung vermieden ist. Einer Kondensatbildung ist bereits dadurch entgegengewirkt, dass die Luftfeuchte nicht in Dampfform eingebracht wird. Sogar kondensatssensible Oberflächen werden durch Zuführen und in Schwebehalten der Luftfeuchte in der beschriebenen Art und Weise, wenn überhaupt nur sehr gering belastet. Dieses wirkt sich kostenreduzierend auf die ansonsten notwendigen Maßnahmen zum Reinhalten derartiger Oberflächen aus.

Vorteilhaft ist des Weiteren, dass die Zuführung der Luftfeuchte als Aerosol in konditionierter Weise erfolgen kann. Dies bedeutet, dass die zugeführten Flüssigkeitströpfchen konditioniert sein können, also: beispielsweise biologische oder andere Zusätze enthalten bzw. als Träger für solche Stoffe dienen können. Eine Zuführung eines beispielsweise mit biologischen Zusätzen ausgerüstetem Aerosols kann vorgenommen werden, um beispielsweise in zeitlichen Abständen einem oder auch mehreren Arbeitsbereichen Fungizide zuzuführen, sollte dieses erforderlich oder sinnvoll sein. Zweckmäßig ist eine solche Maßnahme beispielsweise bei einem Arbeitsbereich, bei dem es sich um eine Reinigungs- oder Waschstation handelt, in der beispielsweise Gebinde gereinigt oder gewaschen werden, welche Gebinde anschließend als Transportbehälter für Lebensmittel dienen. Auf diese Weise kann unter Ausnutzung der besonderen Verbreitung des zugeführten Aerosols innerhalb eines Arbeitsbereiches wirksam einer Schimmelbildung etwa an den Wänden entgegengewirkt werden. Eine solche Arbeitsbereichsbehandlung wird man bevorzugt vornehmen, wenn der Betrieb in diesem Arbeitsbereich ruht.

Zur Konditionierung können auch Duftstoffe zugeführt werden. Dieses bietet sich beispielsweise in Verkaufsräumen zur Unterstützung des Einkaufverhaltens an.

Vorteilhaft ist die Zuführung von Raumfeuchte in Form von Aerosol vor allem auch in der Lebensmittelindustrie, da ein Verpacken in einer solchen Umgebung sich verlängernd auf das mittlere Haltbarkeitsdatum des Lebensmittels auswirken kann. Zudem wirkt ein Lebensmittel, welches in einer solchen Umgebung verpackt worden ist, für Kunden attraktiver.

Besonders vorteilhaft wirkt sich die Luftfeuchtezuführung in Form von Aerosol beispielsweise bei in einem Arbeitsbereich einer Bäckerei befindlichen Teiglinge aus, auf denen sich das Aerosol in einem dünnen Film legt. Verbessert wird hierdurch nicht nur die Qualität des Teiglings und das Backergebnis, sondern, wie vorstehend bereits angedeutet, auch das mittlere Haltbarkeitsdatum, innerhalb dessen der Teigling gebacken werden soll.

Betrieben wird die Lüftungsanlage dergestalt, dass in das Gebäude die Zuluft mit einem gewissen Überdruck eingebracht wird. Infolge dieses Überdruckes bildet sich die gewünschte Luftströmung zu dem oder den Abluftöffnungen aus. Der Druck, mit dem der Zuluftstrom aus dem oder den Luftaustrittskanälen austritt, ist einstellbar. Somit kann in dem Gebäude einer Produktionsstätte der Luftdruck unabhängig von dem in der Umgebung aktuell herrschenden Luftdruck und somit auch zumindest weitgehend unabhängig von wetterbedingten Luftdruckänderungen der Luftdruck konstant gehalten bzw. stärkere Luftdruckschwankungen können gedämpft werden.

Dieses ist zumindest in dem oder den Arbeitsbereichen der Fall, denen Luftaustrittsrohre zugeordnet sind. Auf die Herstellung und Verarbeitung von Teiglingen in Bäckereien hat der Umgebungsdruck und zwar im Zusammenhang mit dem Prozess des Aufgehenlassens Einfluss. Somit kann durch die Steuerbarkeit des Luftdruckes in zumindest einigen Arbeitsbereichen einer solchermaßen als Produktionsstätte ausgelegten Bäckerei, wie dieses Beispiel deutlich macht, eine konstante bzw. zumindest deutlich konstantere Qualität der hergestellten Teiglinge begründen.

Da sich die als Aerosol zugeführte Luftfeuchte nicht als Kondensat niederschlägt, werden Schwebstoffe durch die Flüssigkeitströpfchen gebunden und durch die Luftströmung aus dem reinzuhaltenden Gebäudebereich weggeführt.

Der Konzeption der Lüftungsanlage und der dieser zugeordneten Innenraumbefeuchtungseinrichtung folgend, wird man die Arbeitsbereiche in einer solchen Produktionsstätte einrichten. Diejenigen Arbeitsbereiche, in denen ein möglichst konstanter Luftdruck und eine besondere Sauberkeit herrschen soll, befinden sich unmittelbar im Bereich der Luftaustrittsrohre. Aufgrund der sich einstellenden Luftströmung in dem Gebäude wird man diejenigen Arbeitsbereiche, die in Richtung der Luftströmung zu dem oder den Abluftöffnungen hin angeordnet sind, für Arbeitsvorgänge nutzen, in denen nicht so hohe Anforderungen an Luftdruck und Sauberkeit gestellt sind, als dass diese unmittelbar im Bereich eines oder mehrerer Luftaustrittskanäle sich befinden müssten.

Mit dem vorgeschriebenen Konzept kann nicht nur auf Feuchtigkeitsänderungen im Innenraum rasch und gezielt reagiert werden, sondern mit diesem Konzept kann die Luftfeuchtigkeit in dem gewünschten Maße von 50 bis 55 Prozent gehalten werden. Die Luftfeuchte in dem Gebäude kann, wenn gewünscht, auch auf einen höheren Wert eingestellt werden.

Für den Fall, dass in unterschiedlichen Arbeitsbereichen eine von anderen Arbeitsbereichen unabhängige Zuluftzufuhr über das oder die darin befindlichen Luftaustrittsrohre und eine Aerosolzuführung vorgesehen ist, ist vorzugsweise jedem solchen Arbeitsbereich ein eigenes Förderaggregat, umfassend eine Pumpe für die Zuluftzufuhr sowie einen Nebelgenerator zum Erzeugen des Aerosols, zugeordnet. Auch wenn auf diese Weise für die gesamte Lüftung und Klimatisierung des Gebäudes etliche Förderaggregate benötigt werden, so erlaubt eine solche Auslegung der Produktionsstätte eine besonders unabhängige Regulierung der Raumbedingungen in unterschiedlichen Arbeitsbereichen. Zudem kann in einem solchen Fall auf handelsübliche Aggregate zurückgegriffen werden, so dass die Gesamtkosten einer solchen Anlage nicht aus dem Rahmen fallen.

Die geschickte Auslegung des Gebäudes der Produktionsstätte zum Ermöglichen einer oder mehrerer Luftströmungen und das Nutzen der Prozesswärme einzelner Produktionsmaschinen als Luftströmungsmotor erlaubt einen Betrieb der Lüftungs- und Klimaanlage einer solchen Produktionsstätte mit nur sehr geringen Kosten, verglichen mit den Kosten, die für die Errichtung und den Betrieb herkömmlicher Lüftungs- und Klimatisierungsanlagen benötigt werden.

Zur Luftbefeuchtung wird man typischerweise Wasser, insbesondere steriles Wasser für die Aerosolerzeugung verwenden. Damit die Tröpfchen von dem sich im Innenraum bei einem Betrieb der Lüftungsanlage einstellenden Luftstrom mitgeführt werden können, liegen diese in der Größenordnung typischerweise zwischen 1 bis 3 µm, können jedoch, wenn gewünscht, auch Größen von etwa 20 µm erreichen. Um eine über den zu belüftenden Arbeitsbereich gleichmäßige Belüftung zu erreichen, ist erfindungsgemäß vorgesehen, dass das zumindest eine Luftaustrittsrohr als Textilrohr ausgeführt ist.

Damit die Luftströmung sich innerhalb des Gebäudes der Produktionsstätte ausbilden kann, ist dieses nach Art eines Niedrigenergiehauses hinreichend dicht. Bei Türen und Toren zwischen zwei unterschiedlichen Druckbereichen in dem Gebäude oder auch nach außen hin wird man typischerweise eine Schleuse zwischen den Bereichen unterschiedlichen Druckes vorsehen.

Bevorzugt weist eine solche Produktionsstätte in ihrem Gebäude mehrere Arbeitsbereiche auf, die typischerweise durch Wände voneinander getrennt sind. Dieses ist jedoch nicht unbedingt erforderlich und richtet sich nach den in einem Arbeitsbereich zu verrichtenden Arbeiten. In denjenigen Arbeitsbereichen, in denen erhöhte Anforderungen an einen gleichbleibenden Luftdruck und an Sauberkeit gestellt sind, werden ein oder mehrere Luftaustrittsrohre, typischerweise zusammen mit entsprechenden Aerosolaustrittsrohren angeordnet. In den einzelnen Arbeitsbereichen sind vorzugsweise die Zuluftzufuhr und die Aerosolzufuhr von anderen Arbeitsbereichen mit einer Luft- und Aerosolzufuhr unabhängig einstellbar. Auf diese Weise kann in Bezug auf den in dem Arbeitsbereich gewünschten Luftdruck und die in einem Arbeitsbereich gewünschte Luftfeuchte unabhängig von den Anforderungen in anderen Arbeitsbereichen eingestellt werden. Aufgrund des Lüftungskonzeptes bei einer solchen Produktionsstätte besteht eine Luftströmungswegsamkeit von jedem Arbeitsbereich bis zu der oder den Abluftöffnungen des Gebäudes. Diese befinden sich vorzugsweise am Ende eines Abluftströmungsschachtes, über den die Abluft der oder den Abluftklappen zugeführt wird. Insofern stellt ein solcher Abluftströmungsschacht einen Sammler für die durch den Überdruck in den oder in einzelnen Arbeitsbereichen erzeugte Luftströmung dar. Um dieses zu erreichen, sind Arbeitsbereiche, die durch Wände eingefasst sind, luftströmungstechnisch an einen solchen Abluftstromschacht angeschlossen. Dieses kann beispielsweise realisiert werden, indem die Arbeitsbereiche in einer Produktionsebene in ihren Wänden Strömungsdurchbrechungen aufweisen. Es versteht sich, dass diese hinreichend groß sein müssen, damit sich aufgrund des in aller Regel nur geringen Druckunterschiedes zwischen dem Druck im Bereich der Luftaustrittsrohre und der oder den Abluftöffnungen die gewünschte Luftströmung ausbilden kann. Möglich ist auch eine Luftströmung, bei der diese den jeweiligen Arbeitsbereich nach oben hin verlässt. Bei einer Produktionsstätte mit mehreren Produktionsebenen kann dieses beispielsweise durch Deckendurchbrechungen nach Art von Lufträumen erreicht werden. Es versteht sich, dass auch eine Kombination dieser Maßnahmen zum Realisieren der gewünschten Strömungswegsamkeit genutzt werden können.

Zwischen den einzelnen Arbeitsbereichen, die sich durchaus, wenn in einer Ebene befindlich, durch verschiedene Raumhöhen unterscheiden können, sind diese vorzugsweise durch Strömungsbarrieren voneinander getrennt. Dieses dient dem Ziel, den Luftstromübergang aus dem einen Arbeitsbereich in den anderen Arbeitsbereich zu kontrollieren. Verwirklichen lässt sich dieses beispielsweise von der Decke herabhängende Schotten. Besonders effektiv und vor allem energieeffektiv arbeitet eine solche Entlüftungs- und Klimatisierungsanlage, wenn in dem Gehäuse der Produktionsstätte mehrere Produktionsebenen vorgesehen sind und für den Produktionsprozess ein oder mehrere Prozesswärme abgebende Produktionsmaschinen benötigt werden. Im Falle einer Bäckerei handelt es sich bei den Prozesswärme abgebenden Produktionsmaschinen beispielsweise um die verwendeten Öfen, vor allem wenn als Backöfen Durchlauföfen eingesetzt werden. Für die Luftstromgenerierung bzw. für die Unterstützung des sich allein aufgrund der Lüftungsanlage einstellenden Luftströmung wird man bei einer solchen Produktionsstätte diese Produktionsmaschinen dort aufstellen, wo die Luftströmung oder zumindest ein Teil derselben vorbei strömt. Die durch die abgegebene Prozesswärme bedingte Luftkonvektion unterstützt die in dem Gebäude vorgesehene Luftströmung. Aus diesem Grunde wird man eine solche Prozesswärme abgebende Produktionsmaschine in einem Gebäude mit mehreren Produktionsebenen in einer oberen Produktionsebene nahe oder sogar im Bereich eines Abluftstromschachtes anordnen. Der sich bei einer mit einer solchen Lüftungsanlage ausgerüsteten Produktionsstätte einstellende Kamineffekt wird durch eine solche Maßnahme in nicht unerheblichem Maße verstärkt. Da als Motor der Luftströmung sodann auch eine solche Prozesswärme abgebende Produktionsmaschine zur Verfügung steht, kann, ohne befürchten zu müssen, dass eine Luftströmung zwischen dem bzw. den Luftaustrittsrohren und der bzw. den Abluftöffnungen zum Stillstand kommt, der Zuluftstrom hinsichtlich seines Druckes in einer größeren Bandbreite eingestellt werden. Mithin stellt sich eine Luftströmung auch mit niedrigeren Drücken bzw. Druckdifferenzen zwischen Zuluft und Abluftöffnung ein, bei denen ohne eine Prozesswärme abgebende Produktionsmaschine als Luftstrommotor sich eine den gewünschten Zwecken genügende Luftströmung zwischen dem Luftaustrittsrohr oder den Luftaustrittsrohren und der Abluftöffnung oder den Abluftöffnungen nicht einstellen würde.

In dem oder in einzelnen Arbeitsbereichen wird man in einer bestimmten Höhe ein oder mehrere derartige Textilrohre als Luftaustrittsrohre anordnen. Diese können unterschiedlichen Querschnittsgeometrien und auch unterschiedliche Geometrien in ihrer Längserstreckung aufweisen. Die Geometrien der Längserstreckung wird man an die räumlichen Gegebenheiten anpassen wollen. Durch ein solches Textilrohr tritt die Zuluft durch die Textilwandung hindurch. Dieses erfolgt mit einem gewissen Überdruck mit dem Ergebnis, dass in den zu belüftenden Bereich, in dem der Luftaustritt erfolgt, ein gewisser Überdruck herrscht. Zugeführt wird die Zuluft an das Luftaustrittsrohr oder die Luftaustrittsrohre über Zuluftleitungen. Typischerweise befindet sich das hierzu notwendige Förderaggregat außerhalb des Gebäudes, zumeist auf dem Gebäudedach.

Bei der erfindungsgemäßen Ausgestaltung des Luftaustrittsrohr in Form von einem oder mehreren Textilrohren ist vorgesehen, den Aerosolaustritt ebenfalls als Rohr, nämlich als Aerosolaustrittsrohr auszuführen, und zwar dergestalt, dass sich das Aerosolaustrittsrohr parallel zudem Luftaustrittsrohr erstreckt und in geringem Abstand zu diesem verläuft. Erfindungsgemäß wird man den Aerosolaustritt etwas oberhalb des Zuluftaustrittes anordnen, damit das austretende Aerosol in die sich einstellende Luftströmung, beginnend an dem Zuluftaustritt, schwerkraftbedingt quasi in diese hineinfällt und sodann von der Luftströmung erfasst und nach Art von Schwebfracht mitgeführt wird.

Die zum Betrieb der Lüftungsanlage notwendigen Komponenten sind vorzugsweise sämtlich dem Förderaggregat zugeordnet. Dieses umfasst zumindest die zum Fördern des Zuluftstroms benötigte Luftförderpumpe und den Nebelgenerator. Der Nebelgenerator ist an eine Flüssigkeitsversorgung angeschlossen. In aller Regel werden zusätzliche Luftfilter verwendet, um den Innenraum gefilterte Zuluft zuzuführen. Ist eine Rückgewinnung von Wärme aus dem Abluftstrom vorgesehen, wird man das hierfür benötigte, zumindest eine Wärmerückgewinnungsaggregat ebenfalls dem Förderaggregat zuordnen, sodass sämtliche Elemente in einer Baueinheit zusammen angeordnet sind.

Ein solches, bezüglich der notwendigen Elemente in einem Gehäuse zusammengefasstes Förderaggregat ist gemäß einer bevorzugten Ausgestaltung auf Stützen aufgestellt. Dabei sind die Komponenten eines solchen Förderaggregates in dem Gehäuse angeordnet, dass diese bei Bedarf, insbesondere die Filter, herausgenommen, gereinigt und/oder ausgetauscht werden können. Eine Anordnung des Förderaggregates mit seinem Gehäuse auf Stützen ist vor allem dann vorteilhaft, wenn eine Innenreinigung der einzelnen Aggregate und/oder des Gehäuses flüssigkeitsbasiert vorgesehen ist. Dann wird man das Gehäuse in einer Wanne vorsehen oder bodenseitig eine Flüssigkeitsaufnahmerinne umlaufend um den Boden des Gehäuses vorsehen. Beides dient zur Aufnahme der Reinigungsflüssigkeit. Damit diese nicht wahllos aus der Flüssigkeitsaufnahmerinne oder der Wanne ausläuft, sind ein oder mehrere Abflussöffnungen vorgesehen. Aus diesen kann das Reinigungswasser austreten und, sollte sich das Förderaggregat auf dem Dach eines Gebäudes befinden, der Dachentwässerung zugeführt werden. Möglich ist es bei einer solchen Ausgestaltung auch, an die zumindest eine Abflussöffnung eine Leitung, etwa ein Rohr oder einen Schlauch anzuschließen, durch das beziehungsweise die das Reinigungsabwasser entsorgt wird, beispielsweise einer Reinigungswasseraufbereitung zugeführt wird.

Eine solche Produktionsstätte wird man vorzugsweise mit möglichst homogener und mit einem niedrigen Wärmedurchgangskoeffizienten versehener Oberfläche nach außen und daher prinzipiell unter Anwendung des so genannten Niedrigenergiehausstandards auslegen und anfallende Prozesswärme für eine Erwärmung der Zuluft, falls notwendig, und/oder für andere Wärme benötigende Produktionsprozesse nutzen. Die Auslegung einer Produktionsstätte mit einem solchen Standard ist somit dergestalt vorgesehen, dass die geforderten energietechnischen Anforderungsniveaus in der Summe unterschritten werden. Gemäß einem bevorzugten Ausführungsbeispiel wird der oder es werden die sich einstellenden Luftströmungen in Bezug auf ihre jeweilige Temperatur überwacht. Zudem wird denjenigen Arbeitsbereichen, in denen ein möglichst konstanter Luftdruck herrschen soll, der Luftdruck überwacht. Zum Zwecke einer Wärmebilanzierung wird ebenfalls die Abluftstrommenge und deren Temperatur überwacht. Die Daten der hierfür eingesetzten Sensoren beaufschlagen eine zentrale Steuereinheit, die dann wiederum zum Nachregeln von Schwankungen in Bezug auf die gewünschte Temperatur, den gewünschten Luftfeuchtigkeitsgehalt und den gewünschten Luftdruck den oder die entsprechenden Aktoren entsprechend ansteuert.

Im Rahmen des Betriebs einer solchen Lüftungsanlage, vor allem in Bereichen mit einer größeren Anzahl an Personen, wie etwa in Schulungs-, Büro- oder Kantinenräumen kann mit Frischluftzufuhr gekühlt werden, um auf diese Weise den CO₂-Gehalt zu reduzieren, was einem zu raschen Ermüden entgegenwirkt.

Die Leitungen und insbesondere die Aerosolzuleitungen werden typischerweise regelmäßig entkeimt, beispielsweise mit Ozon.

Um einer Kondensatansammlung in der Aerosolleitung, sollte dieses tatsächlich eintreten, entgegenzuwirken, können diese Leitungen mit einem gewissen Gefälle verlegt werden.

Besonders effektiv lässt sich eine solche Produktionsstätte betreiben, wenn diese ausgelegt ist, eine gewisse Speicherkapazität für die Klimatisierungsvariablen Temperatur, Feuchte und Druck aufweist. Bezüglich der Raumluftfeuchtigkeit kann dieses durch Bereitstellen einer gewissen Speichermasse, etwa über die Wände erreicht werden. Bezüglich der Druckdichtigkeit kann dieses durch Schotte, Schleusen, oder dergleichen neben einer bereits beschriebenen Außenabdichtung verwirklicht werden. Als Temperaturpuffer können ebenfalls die Wände und auch in einem Arbeitsbereich befindliche Gegenstände dienen. Im Zusammenhang mit einer bezüglich dieser Klimatisierungsvariablen vorgesehenen Steuerung des oder der Arbeitsbereiche der Produktionsstätte wird man die Be- und Entlüftung kontrollieren.

Nachfolgend ist die Erfindung anhand eines Ausführungsbeispiels unter Bezugnahme auf die beigefügten Figuren beschrieben. Es zeigen:
- **Fig. 1:**: in einem vertikalen Schnitt ein Gebäude mit einer Lüftungsanlage zum Erläutern des beanspruchten Konzeptes und
- **Fig. 2:**: einen vertikalen Schnitt durch ein Gebäude einer Produktionsstätte mit einer nach den Prinzipien der Lüftungsanlage der Figur 1 arbeitenden Lüftungs- und Klimatisierungsanlage.

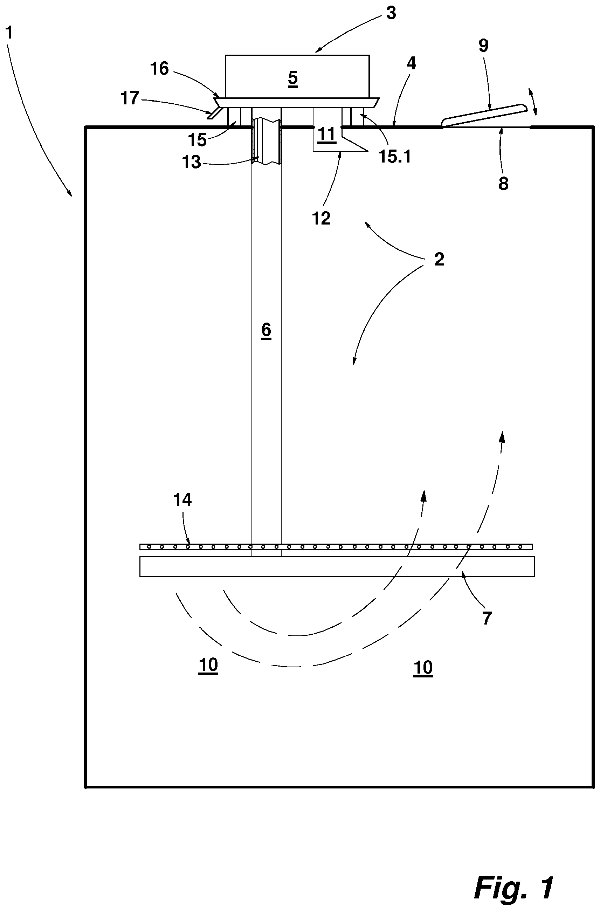

Zunächst wird das Lüftungs- und Klimatisierungsprinzip, wie dieses bei der beanspruchten Produktionsstätte eingesetzt wird, anhand der Figur 1 erläutert. In Figur 1 ist in einem vertikalen Schnitt ein Gebäude 1 mit einer Lüftungsanlage 2 dargestellt. Die Lüftungsanlage 2 umfasst ein Förderaggregat 3, welches auf dem Dach 4 des Gebäudes 1 aufgestellt ist. Die einzelnen Teile des Förderaggregats 3 sind in einem gemeinsamen Gehäuse 5 angeordnet und in der Figur 1 nicht näher dargestellt. Das Förderaggregat umfasst eine Luftförderpumpe, durch die Zuluft in eine Zuluftleitung 6 gefördert wird. Das Förderaggregat 3 umfasst des Weiteren zumindest einen Filter, durch den die aus der Umgebung entnommene Zuluft vor Einbringen derselben in die Zuluftleitung 6 gefiltert wird. Durch die Zuluftleitung 6 ist das Förderaggregat 3 beziehungsweise die darin enthaltene Luftförderpumpe mit einem Luftaustrittsrohr 7 verbunden. Bei dem Luftaustrittsrohr 7 des dargestellten Ausführungsbeispiels handelt es sich um ein an sich bekanntes Textilrohr. Aus diesem wird die in die Zuluftleitung 6 geförderte Zuluft aufgrund der porösen Eigenschaften der Wandung heraus gedrückt.

Der Lüftungsanlage 1 ist zumindest eine Abluftöffnung 8 zugeordnet. Diese ist bei dem dargestellten Ausführungsbeispiel durch eine Abluftklappe 9 realisiert, die hinsichtlich ihrer Öffnungsweite und damit hinsichtlich ihrer freien durchströmbaren Querschnittsfläche einstellbar ist. Es versteht sich, dass die Abluftklappe 9 nur beispielhaft als Glied zum Einstellen der Öffnungsweite der Abluftöffnung 8 gezeigt ist. An dieser Stelle können auch Lamellen oder andere die Öffnungsweite der Abluftöffnung ändernde Stellglieder eingesetzt werden. Auch muss sich die Abluftöffnung 8 nicht, wie bei dem dargestellten Ausführungsbeispiel gezeigt, in dem Dach 4 des Gebäudes befinden, sondern kann ebenfalls an ein oder mehreren Seitenwänden desselben angeordnet sein. In Abhängigkeit von der Stellung der Abluftklappe 9 ist die Abluftöffnung 8 größer oder kleiner, sodass durch die Stellung der Abluftklappe 9 der an die Umgebung abgegebene Abluftstrom einrichtbar ist. Die Verstellbarkeit der Abluftklappe 9, die bei dem dargestellten Ausführungsbeispiel durch einen elektrischen Stellmotor bewirkt wird, ist durch den benachbart zu dieser in der Figur 1 eingetragenem Doppelpfeil kenntlich gemacht.

Das Förderaggregat 3 verfügt zudem über eine Rezirkulationswegsamkeit, über die Abluft durch das Förderaggregat 3 hingeführt werden kann. Eine solche Wegsamkeit kann ausgebildet sein, Abluft dem Zuluftstrom zuzuführen und/oder um Wärme aus dem Abluftstrudel eines Wärmetauscheraggregates zu entnehmen und dem Zuluftstrom zuzuführen. Letzteres ist vor allem dann zweckmäßig, wenn in dem Innenraum 10 des Gebäudes Prozesswärme entsteht und abzuführen ist. Dann kann diese genutzt werden, um beispielsweise in Wintermonaten kalte Zuluft aus der Umgebung zu erwärmen. Eine solche Wärmerückgewinnung wirkt sich positiv auf die Energiebilanz bei einem Betrieb der Lüftungsanlage und somit bezüglich der Betriebskosten des Gebäudes 1 aus. Zum Einleiten eines solchen rezirkulierenden Abluftstromes ist dem Förderaggregat 3 Abluftstutzen 11 zugeordnet, der mit seiner Öffnung 12 etwas unterhalb der Decke 4 des Gebäudes 1 angeordnet ist. In Abhängigkeit von der Stellung der Abluftklappe 9 kann der in den Abluftstutzen 11 eintretende Abluftstrom beziehungsweise Abluftstromanteil eingestellt werden.

Das Förderaggregat 3 umfasst des Weiteren einen Nebelgenerator zum Erzeugen eines Aerosols. Das Aerosol wird über eine Aerosolzuleitung 13 einem in den Innenraum 10 angeordneten Aerosolaustrittsrohr 14 zugeführt. Bei dem dargestellten Ausführungsbeispiel ist die Aerosolzuleitung 13 innerhalb der Zuluftleitung 6 angeordnet und in nicht dargestellter Art und Weise aus der Zuluftleitung 6 in Höhe des Aerosolsaustrittsrohrs 14 herausgeführt. Das Aerosolaustrittsrohr 14 verfügt über Öffnungen, aus denen das zugeführte Aerosol austritt. Das Aerosolaustrittsrohr 14 befindet sich in Bezug auf die Höhe des Innenraums 10 mit geringem Abstand oberhalb des als Textilrohr ausgeführten Luftaustrittsrohrs 7. Das austretende Aerosol fällt sodann schwerkraftbedingt an dem Luftaustrittsrohr 7 vorbei in die sich zwischen Luftaustrittsrohr 7 und der Abluftöffnung 8 und/oder dem Abluftstutzen 11 einstellende Luftströmung. Die durch den Nebelgenerator erzeugte Tröpfchengröße ist hinreichend klein, damit die Aerosoltröpfchen von dieser Luftströmung als Schwebfracht mitgeführt werden. Die sich einstellende Luftströmung ist in Figur 1 im Bereich des linken Teils des Luftaustrittsrohrs 7 schematisiert angedeutet. Durch diese schematisierte Darstellung der Luftströmung soll gezeigt werden, dass diese durch den zu belüftenden Innenraum 10 des Gebäudes 1 hindurchgeführt wird und dann nach oben in Richtung zur Abluftöffnung 8 aufsteigt. Insofern befindet sich das Luftaustrittsrohr 7 in Bezug auf die Höhe des Gebäudes 1 auf einem deutlich tieferen Niveau als die Abluftöffnung 8.

Eine Zuführung der Luftfeuchte in Form von kleinsten Flüssigkeitströpfchen eines Aerosols hat nicht nur eine Zuführung von Luftfeuchte in den Innenraum 10 zur Folge. Vielmehr führt die Zuführung der Luftfeuchte in dieser Form zugleich zu einer adiabatischen Kühlung. In dem Innenraum 8 entstehende Prozesswärme wird somit nicht nur durch den Abluftstrom abgeführt, sondern zugleich gekühlt. Entsprechend gering ist der Aufwand, für den Fall, dass der Innenraum 10 zusätzlich klimatisiert, also: gekühlt, werden müsste.

Das in dem Gehäuse 5 befindliche Förderaggregat 3 ist auf dem Dach 4 des Gebäudes 1 auf Stützen 15, 15.1 aufgestellt. Das Gehäuse 5 ist in einer Flüssigkeitsauffangwanne 16 angeordnet. Die Wanne 16 verfügt über eine Ablauföffnung, an die ein Ablaufrohrstück 17 angeschlossen ist. Das Vorsehen der Wanne 16 und des Ablaufrohrstückes 17 dienen dem Zweck, Reinigungsflüssigkeit, die vor allem bei der Innenreinigung des Gehäuses 5 beziehungsweise darin vorhandener Aggregate anfällt, zielgerichtet der ohnehin vorhandenen Dachentwässerung zuzuführen.

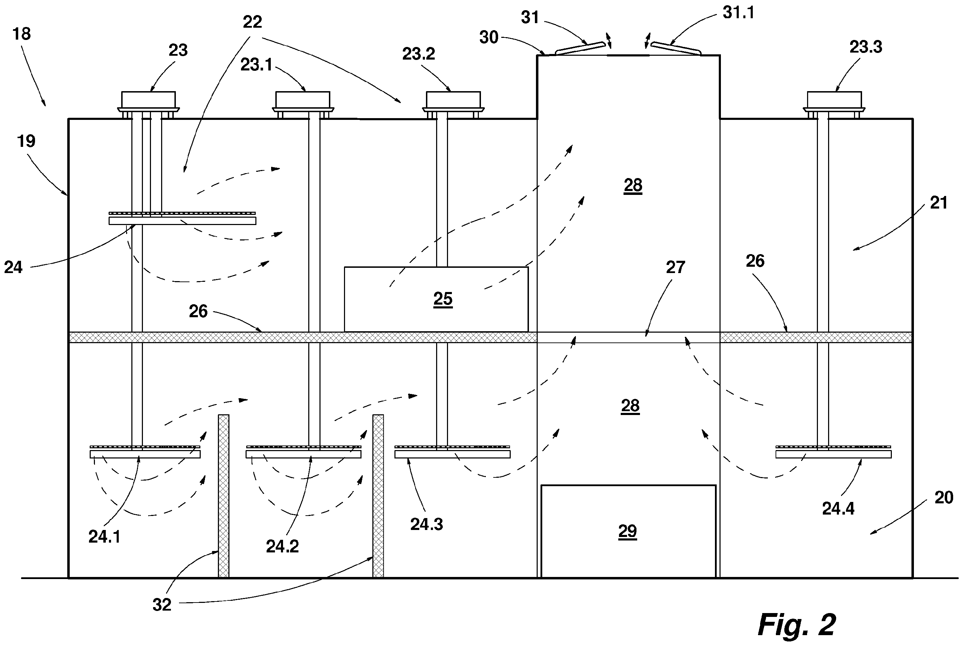

Figur 2 zeigt eine Produktionsstätte 18 mit einem mehrgeschossigen Gebäude 19, wobei bei dem dargestellten Ausführungsbeispiel zwei Produktionsetagen 20, 21 vorgesehen sind. Bei der Produktionsstätte 18 handelt es sich um eine Bäckerei mit integrierter Konditorei. Neben dem Gebäude 19 verfügt die Produktionsstätte 18 über eine Lüftungsanlage 22, die aufgebaut ist, wie die Lüftungsanlage 2 des Gebäudes 1. Die Lüftungsanlage 22 umfasst mehrere Förderaggregate 23, 23.1, 23.2, 23.3, die über Zuluftleitungen jeweils ein Luftaustrittsrohr mit zumindest einem diesem zugeordneten Aerosolaustrittsrohr mit Zuluft und Aerosol beaufschlagen. Diese Einheiten der Lüftungsanlage 22 sind der Übersicht halber in der Produktionsstätte 18 im Unterschied zu der Lüftungsanlage 2 nur mit ihren für die Beschreibung der beanspruchten Erfindung zum Verständnis notwendigen Bestandteilen gezeigt. Die Förderaggregate 23, 23.1, 23.2, 23.3 sind auf dem Dach des Gebäudes 19 angeordnet. Bei der Produktionsstätte 18 umfasst die Lüftungsanlage 22 mehrere Förderaggregate 23 bis 23.3, damit in unterschiedlichen Arbeitsbereichen für eine unterschiedliche und von den übrigen Arbeitsbereichen unabhängig steuerbares Arbeitsbereichsklima eingestellt werden kann. Somit befinden sich die Luftaustrittsrohre nebst Aerosolaustrittsrohren der einzelnen Förderaggregate 23 bis 23.3 in unterschiedlichen Arbeitsbereichen und auch in unterschiedlichen Produktionsetagen 20, 21. In dem Gebäude 19 sind die jeweiligen Luftaustrittsrohre zusammen mit den jeweiligen Aerosolaustrittsrohren mit den Bezugszeichen 24 bis 24.4 kenntlich gemacht. Während sich die Luft- und Aerosolaustrittsrohre 24, die von dem Förderaggregat 23 beaufschlagt sind, sich in der oberen der beiden Produktionsetagen 20, 21 befindet, sind die übrigen Luft- und Aerosolaustrittsrohre 24.1 in der unteren Produktionsetage 20 angeordnet. Die Luft- und Aerosolaustrittsrohre 24 bis 24.4, befinden sich in jeweils unterschiedlichen Arbeitsbereichen des Gebäudes 19 in einer Höhe, dass durch diese die Produktion und der Flurverkehr nicht gestört sind.

Von dem Förderaggregat 23 sind gleichermaßen die Luft- und Aerosolaustrittsrohre 24 aus der oberen Produktionsetage 20 und die Luft- und Aerosolaustrittsrohre 24.1 aus einem Arbeitsbereich in der unteren Produktionsetage 20 beaufschlagt. Dieses erfolgt vor dem Hintergrund, dass bei dem dargestellten Ausführungsbeispiel diese beiden Arbeitsbereiche eine gleiche Klimatisierung und einen gleichen Arbeitsbereichdruck aufweisen sollen. Der den Luft- und Aerosolaustrittsrohren 24 zugeordnete Arbeitsbereich dient einer Backvorbereitung. In diesem Arbeitsbereich werden Teiglinge erstellt und zum Aufgehen gelagert, bevor diese in einen als Durchlaufofen konzipierten Backofen 25 eingebracht werden. Auch der Backofen 25 befindet sich in der oberen Produktionsetage 21, benachbart zu dem eben beschriebenen Arbeitsbereich. Die die beiden Produktionsetagen 20, 21 trennende Geschossdecke 26 ist in einem Teilbereich zur Ausbildung eines Luftraumes durchbrochen. Diese Durchbrechung 27 dient zum Bereitstellen einer Luftströmungswegsamkeit von der unteren Produktionsetage 20 in die obere Produktionsetage 21. Ein Fahrstuhlschacht 28, dessen Fahrstuhl 29 sich in der Darstellung der Figur 2 in der unteren Produktionsetage 20 befindet, dient bei diesem Ausführungsbeispiel als Sammelschacht, um die erzeugten Luftströmungen zu sammeln und als Abluft aus dem Dach 30 des Fahrstuhlschachtes 28 auszulassen. Mehrere Abluftklappen 31, 31.1 sind hinsichtlich ihrer Öffnungsweite ebenso einstellbar wie die Abluftklappe 9 der Lüftungsanlage 1. In Abhängigkeit von der Einstellung der Öffnungsweite der Abluftklappen 31, 31.1 werden die in dem Gebäude 19 generierten Luftströmungen beeinflusst.

Nicht dargestellt ist in Figur 2 eine Wärmerückgewinnung aus dem Abluftstrom, welche Wärme genutzt wird, um bedarfsweise die zugeführte Zuluft zu erwärmen und/oder Wärme benötigenden Produktionsschritten zugeführt wird. Diesbezüglich kann eine Rückleitung erfolgen, wie dieses prinzipiell zu der Lüftungsanlage 2 der Figur 1 beschrieben ist.

Die in der unteren Produktionsetage 20 befindlichen Luft- und Aerosolaustrittsrohre 24.1, 24.2, 24.3, 24.4 erzeugen jeweils eine Luftströmung nach den zuvor beschriebenen Prinzipien. In Figur 2 sind die sich bei einem Betrieb der Lüftungsanlage 2 einstellenden Luftströmungen schematisiert durch gestrichelte Pfeile dargestellt. Die den Luft- und Aerosolaustrittsrohren 24.1 bis 24.4 jeweils zugeordneten Arbeitsbereiche sind durch Wände 32 voneinander getrennt. Bei dem dargestellten Ausführungsbeispiel erstrecken sich die Wände 32 nicht bis an die Geschossdecke 26. Der verbleibende Zwischenraum wird als Strömungsdurchbrechung genutzt, damit sich von jedem der zusammengefassten Luft- und Aerosolaustrittsrohre 24.1 bis 24.4 eine Luftströmung zu dem als Abluftsammelschacht genutzten Fahrstuhlschacht 28 bei einem Betrieb der Lüftungsanlage 2 ausbildet. Bei dem dargestellten Ausführungsbeispiel dienen die Arbeitsbereiche der Luft- und Aerosolaustrittsrohre 24.1, 24.2 einer Arbeitsvorbereitung. Der Arbeitsbereich mit dem Luft- und Aerosolaustrittsrohr 24.3 ist als Konditorei ausgeführt. Auf der anderen Seite des Fahrstuhlschachtes 28 befindet sich ein Arbeitsbereich, in dem die Luft- und Aerosolaustrittsrohre 24.4 angeordnet sind. Dieser Arbeitsbereich ist als Spül- und Reinigungsbereich eingerichtet. In diesem werden Transportgebinde, typischerweise Kunststoffkästen nach ihrer Benutzung gespült, um sodann wieder in dem Produktionsprozess verwendet werden zu können.

Bei einem Betrieb des Ofens 25 setzt dieser eine nicht unerhebliche Menge an Prozesswärme frei. Diese ist bestrebt aufzusteigen, und zwar in den Fahrstuhlschacht 28 hinein. Infolge der einsetzenden Konvektion entsteht in den kühleren Bereichen, und zwar auch in der unteren Produktionsetage 20 sowie in dem Arbeitsbereich, in den die Luft- und Aerosolaustrittsrohre 24 münden, ein gewisser Sog. Infolgedessen fungiert der Ofen 25 bei Betrieb auch als Luftströmungsmotor. Bei der Produktionsstätte 18 handelt es sich um eine solche, die prinzipiell 24 Stunden am Tag arbeitet. Daher befindet sich der als Luftströmungsmotor eingesetzte Ofen 25 ununterbrochen oder quasi ununterbrochen in Betrieb.

Die sich in dem Gebäude 19 einstellende Luftströmungen, ausgehend von jedem Luftaustrittsrohr, erstrecken sich, wie aus Figur 2 ersichtlich, zunächst vornehmlich in horizontaler Richtung in Richtung zu dem als Sammelschacht dienenden Fahrstuhlschacht 28 und sodann in vertikaler Richtung nach oben zu den Abluftklappen 31, 31.1.

Durch jedes Förderaggregat 23 bis 23.3 können die von diesem jeweils beaufschlagten Luft- und Aerosolaustrittsrohre unabhängig angesteuert werden. Dieses ermöglicht die Einstellung eines unterschiedlich starken Überdruckes im Bereich der Luftaustrittsrohre in jedem Arbeitsbereich, der durch ein Förderaggregat 23 bis 23.3 beaufschlagt ist, sowie eine Einstellung einer unabhängigen Arbeitsbereichluftfeuchte ebenso wie eine Arbeitsbereich unterschiedliche Umgebungsdruckeinstellung.

Es versteht sich, dass für die beschriebene Betriebsweise der Lüftungsanlagen 22 das Gebäude 19 nach Art eines Niedrigenergiehauses nach außen hin strömungstechnisch hinreichend dicht ist.

Die Zuführung der Arbeitsbereichluftfeuchte in Form von Aerosol ermöglicht es, dieses als Träger zum Zuführen von biologischen Stoffen in einem Arbeitsbereich zu nutzen. Dieses wird bei dem dargestellten Ausführungsbeispiel über das Förderaggregat 23.3 zum Ausbringen von biologisch aktiviertem Aerosol aus dem Aerosolaustrittsrohr der Luft- und Aerosolaustrittsrohre 24.4 genutzt. Diese Luft- und Aerosolaustrittsrohre 24.4 befinden sich im Arbeitsbereich "Spülen". Aufgrund der hohen Luftfeuchte besteht grundsätzlich die Gefahr einer Versottung. In zeitlichen Abständen wird das diesem Arbeitsbereich zugeführte Aerosol mit einem Fungizid beaufschlagt, und zwar typischerweise dann, wenn der Spülprozess ruht. Aus diesem Grunde ist der Spülarbeitsbereich auch von dem als Abluftstromsammler dienenden Fahrstuhlschacht 28 von den Lebensmittel ver- und bearbeitenden Arbeitsbereichen getrennt.

Die Erfindung ist anhand eines Ausführungsbeispieles einer Produktionsstätte beschrieben worden. Ohne den Umfang der geltenden Ansprüche zu verlassen, kann das beschriebene Konzept auch auf andere Weise ausgeführt werden.

### Bezugszeichenliste

- 1: Gebäude
- 2: Lüftungsanlage
- 3: Förderaggregat
- 4: Dach
- 5: Gehäuse
- 6: Zuluftleitung
- 7: Luftaustrittsrohr
- 8: Abluftöffnung
- 9: Abluftklappe
- 10: Innenraum
- 11: Abluftstutzen
- 12: Öffnung
- 13: Aerosolzuleitung
- 14: Aerosolaustrittsrohr
- 15, 15.1: Stütze
- 16: Wanne
- 17: Ablaufrohrstück
- 18: Produktionsstätte
- 19: Gebäude
- 20: Produktionsetage
- 21: Produktionsetage
- 22: Lüftungsanlage
- 23, 23.1, 23.2, 23.3: Förderaggregat
- 24, 24.1, 24.2, 24.3, 24.4: Luft- und Aerosolaustrittsrohre
- 25: Ofen
- 26: Geschossdecke
- 27: Durchbrechung
- 28: Fahrstuhlschacht
- 29: Fahrstuhl
- 30: Dach
- 31,31.1: Abluftklappe
- 32: Wand

## Patentansprüche

1. Produktionsstätte umfassend
- ein geschlossenes Gebäude (19), in dem ein, vorzugsweise mehrere Arbeitsbereiche angeordnet sind,
- eine Lüftungsanlage (22)
a) mit wenigstens einem Förderaggregat zum Fördern von Frischluft mittels eines Zuluftstromes in das Gebäude (19) hinein, welches zumindest eine Förderaggregat (23 - 23.3) wenigstens einen in dem Gebäude (19) angeordneten, als Textilrohr ausgebildetes Luftaustrittsrohr (7), über eine Zuluftleitung (6) mit dem Förderaggregat (23 - 23.3) verbunden, mit einem Zuluftstrom beaufschlagt, und
b) mit einer einstellbaren Abluftöffnung (31, 31.1), durch die Abluft aus dem Gebäude (19) austreten kann,
c) wobei das zumindest eine Luftaustrittsrohr (7) mit Abstand zu der zumindest einen Abluftöffnung (31, 31.1) dergestalt angeordnet ist, dass sich bei einem Betrieb der Lüftungsanlage (22) in dem Gebäude (19) eine Luftströmung von dem zumindest einen Luftaustrittsrohr (7) zu der zumindest einen Abluftöffnung (31, 31.1) einstellt,
- eine der Lüftungsanlage (22) zugeordnete Befeuchtungseinrichtung zum Zuführen von Luftfeuchte in das Gebäude (19), welche Befeuchtungseinrichtung wenigstens einen in dem Förderaggregat (23 - 23.1) angeordneten Ultraschallvernebler als Nebelgenerator zum Erzeugen eines Aerosols aus einer Flüssigkeit mit Flüssigkeitströpfchen in einer Größe, damit diese von der sich in dem Gebäude (19) einstellende Luftströmung mitgeführt werden können, und zumindest ein Aerosolaustrittsrohr (14) umfasst, das sich parallel zu dem Luftaustrittsrohr (7) erstreckt und in geringem Abstand oberhalb zu diesem verläuft, damit das austretende Aerosol von der Luftströmung erfasst wird,
- eine Aerosolzuleitung (6), über welche das Aerosol dem zumindest einen Aerosolaustrittsrohr (13) zugeführt wird, und
- Arbeitsbereiche, die in Richtung der Luftströmung zu der oder den Abluftöffnungen hin angeordnet sind, wobei ein Arbeitsbereich, in dem ein konstanter Luftdruck und Sauberkeit herrschen soll, sich unmittelbar im Bereich des Luftaustrittsrohres (7) befindet.

2. Produktionsstätte nach Anspruch 1, wobei das Gebäude (19) durch Vorsehen von zumindest zwei Produktionsebenen (20, 21) mehrgeschossig ist, wobei die Geschosse (20, 21) durch wenigstens einen Strömungsdurchgang (27) zum Durchlassen der durch die Lüftungsanlage (22) erzeugten Luftströmung miteinander verbunden sind, wobei ein oder mehrere Arbeitsbereiche in der unteren Produktionsebene (20) und ein oder mehrere weitere Arbeitsbereiche in der darüber befindlichen Produktionsebene vorgesehen sind und wobei das mindestens eine Luftaustrittsrohr (7) in der unteren Produktionsebene (20) angeordnet ist.

3. Produktionsstätte nach Anspruch 1, wobei zumindest eine Produktionsebene (20), in denen Luftaustrittsrohre (7) angeordnet sind, in durch Wände (32) begrenzte Arbeitsbereiche gegliedert ist und in zumindest zwei Arbeitsbereichen jeweils wenigstens ein unabhängig von dem oder den in anderen Arbeitsbereichen angeordneten Luftaustrittsrohren (7) mit einem Zuluftstrom beaufschlagbares Luftaustrittsrohr (7) mit einem diesem zugeordneten Aerosolaustrittsrohr (13) angeordnet ist.

4. Produktionsstätte nach Anspruch 2 oder 3, wobei die Produktionsebenen (20, 21) durch wenigstens einen Strömungsdurchgang (27) zwischen den Geschossen zum Durchlassen der durch die Lüftungsanlage (22) erzeugten Luftströmung miteinander verbunden sind.

5. Produktionsstätte nach einem der Ansprüche 2 bis 4, wobei die Produktionsebene (20), in der die Luftaustrittsrohre (7) angeordnet sind, in durch Wände (32) begrenzte Arbeitsbereiche gegliedert ist, die untereinander durch wenigstens einen Strömungsdurchgang zum Durchlassen des durch die Lüftungsanlage erzeugten Luftströmung miteinander verbunden sind.

6. Produktionsstätte nach einem der Ansprüche 2 bis 5, wobei in wenigstens einem Arbeitsbereich eine Prozesswärme abgebende Produktionsmaschine, etwa ein Ofen (25), angeordnet ist und sich dieser Arbeitsbereich in einer Produktionsebene (21) oberhalb derjenigen Produktionsebene (20) befindet, in der Luftaustrittsrohre (7) angeordnet sind.

7. Produktionsstätte nach Anspruch 6, wobei die Prozesswärme abgebende Produktionsmaschine (25) innerhalb der Produktionsebene (21) dergestalt positioniert ist, damit die durch die Lüftungsanlage (22) erzeugte Luftströmung zumindest teilweise an dieser vorbei strömt.

8. Verfahren zum Klimatisieren eines geschlossenen Gebäudes (19) einer Produktionsstätte (18), wobei durch wenigstens ein Luftaustrittsrohr (7) ein Zuluftstrom in das Gebäude (19) hinein mit einem dem an dieser Stelle ohne Zuluftstrom herrschenden Druck höheren Druck eingebracht wird, welcher Zuluftstrom durch das Gebäude (19) oder einen Teil desselben zu wenigstens einer Abluftöffnung (31, 31.1) geleitet wird, Luftaustrittsrohr (7) ein Aerosol mit Flüssigkeitstropfen in einer Größe beigemengt wird, damit diese von der sich in dem Gebäude (19) einstellenden Luftströmung mitgeführt werden, wbei das Aerosol von einem in einer Luftförderungseinrichtung angeordnetem Ultraschallvernebler über eine Aerosolzuleitung (13) dem Aerosolaustrittsrohr (14) zugeleitet wird, welches Aerosolaustrittsrohr (14) sich parallel zu dem Luftaustrittsrohr (7) erstreckt und in geringem Abstand oberhalb zu diesem verläuft, wobei die Aerosol beladene Luftströmung auf ihrem Weg zu zumindest einer Abluftöffnung (31, 31.1) des Gebäudes (19) durch Arbeitsbereiche, die in Richtung zu der Luftströmung zu der oder den Abluftöffnungen hin angeordnet sind geführt wird, wobei ein Arbeitsbereich, in dem ein konstanter Luftdruck und Sauberkeit herrschen soll, sich unmittelbar im Bereich eines Luftaustrittsrohres (7) befindet.

9. Verfahren nach Anspruch 8, wobei das zu klimatisierende Gebäude (19) zumindest zwei Produktionsebenen (20, 21) aufweist und die sich durch einen Betrieb der Lüftungsanlage (22) einstellende Luftströmung zumindest teilweise das Gebäude (19) in vertikaler Richtung durchströmt.

10. Verfahren nach Anspruch 9, wobei die Luftstrombewegung in dem Gebäude (19) zumindest in einem ihrer vertikal strömenden Abschnitte durch ein oder mehrere Prozesswärme abgegebene Produktionsmaschinen (25) unterstützt oder erst ermöglicht wird.

11. Verfahren nach einem der Ansprüche 8 bis 10, wobei die Zuluft und das Aerosol in einer Produktionsebene (20) in mehreren Arbeitsbereichen, die durch Wände voneinander getrennt sind, zugeführt wird, wobei eine Zuführung der Zuluft und/oder des Aerosols in einzelnen Arbeitsbereichen unabhängig von der Zuluft- und/oder Aerosolzuführung in anderen Arbeitsbereichen eingestellt wird.

12. Verfahren nach einem der Ansprüche 8 bis 10, wobei das zuzuführende Aerosol mit einem biologischen Wirkstoff angereichert wird.

## Claims

1. Production site comprising
- a closed building (19), arranged in which are preferably several working areas,
- a ventilation system (22)
a) with at least one conveying apparatus for conveying fresh air by means of a delivery stream into the building (19), which at least one conveying apparatus (23 - 23.3) comprises at least one air outlet pipe (7), arranged in the building (19) and configured as a textile pipe, and is connected to the conveying apparatus (23 - 23.3) via a delivery line (6), and is subjected to a delivery air flow, and
b) with an adjustable discharge air opening (31, 31.1), through which the discharge air can leave the building (19),
c) wherein the at least one air outlet pipe (7) is arranged at a distance interval from the at least one discharge air opening (31, 31.1) is arranged in such a way that, during operation of the ventilation system (22), an air flow is introduced in the building (19) from the at least one air outlet pipe (7) to the at least one discharge air opening (31, 31.1),
- a humidifying device assigned to the ventilation system (22) for delivering air humidity into the building (19), which humidifying device comprises at least one ultrasonic nebulizer arranged in the conveying apparatus (23 - 23.1) as a mist generator for producing an aerosol from a fluid with fluid droplets in a size such as can be conveyed with the air flow introduced in the building (19), and at least one aerosol outlet pipe (14), which extends parallel to the air outlet pipe (7) and runs above it with a slight distance interval, in order for the emerging aerosol to be taken up by the air flow,
- an aerosol line (6), by means of which the aerosol is conveyed to the at least one aerosol discharge pipe (13), and
- working areas, which are arranged in the direction of the air flow towards the discharge air openings, wherein a working area, in which it is intended that a constant air pressure and cleanliness should be maintained, is located directly in the region of the air discharge pipe (7).

2. Production site according to claim 1, wherein the building (19) is multi-storey by the provision of at least two production levels (20, 21), wherein the storeys (20, 21) are connected to one another by at least one flow passage (27) for allowing the passage of the air flow produced by the ventilation system (22), wherein one or more working areas are provided in the lower production level (20) and one or more working areas are provided in the production level located above this, and wherein the at least one air discharge pipe (7) is arranged in the lower production level (20).

3. Production site according to claim 1, wherein at least one production level (20), in which air outlet pipes (7) are arranged, is divided up into working areas delimited by walls (32), and in at least two working areas there is arranged in each case at least one air outlet pipe (7), arranged independently from the other air discharge pipe(s) (7) arranged in other working areas, which can be subjected to an inlet air flow, and with an aerosol discharge pipe (13) assigned to it.

4. Production site according to claim 2 or 3, wherein the production levels (20, 21) are connected to one another by at least one flow passage (27) between the storeys, for allowing the passage of the air flow produced by the ventilation system (22).

5. Production site according to any one of claims 2 to 4, wherein the production level (20), in which the air outlet pipes (7) are arranged, is divided into working areas delimited by walls (32), which are connected to one another by at least one flow passage for allowing the passage of the air flow produced by the ventilation system.

6. Production site according to any one of claims 2 to 5, wherein arranged in at least one working area is a production machine (25) emitting process heat, such as an oven, and this working area is located on a production level (21) above that production level (20) in which air outlet pipes (7) are arranged.

7. Production site according to claim 6, wherein the production machine (25) emitting process heat is positioned inside the production level (21) in such a way that the air flow produced by the ventilation system (22) flows at least in part past this.

8. Method for air-conditioning a closed building (19) of a production site (18) wherein an inflow air stream is introduced into the building (19) through at least one air outlet pipe (7), at a pressure which is higher than the pressure prevailing at this location, which inflow air stream is conducted through the building (19) or a part thereof to at least one outlet air opening (31, 31.1), an aerosol is mixed in the air outlet pipe (7) with fluid droplets in a size such that they can be conveyed with the air flow introduced in the building (19), wherein the aerosol is conveyed from an ultrasonic nebulizer arranged in an air conveying apparatus, via an aerosol delivery line (13) to the aerosol outlet pipe (14), which aerosol outlet pipe (14) extends parallel to the air outlet pipe (7) and runs above it with a slight distance interval,
wherein the aerosol-laden air flow is conveyed, on its way to at least one air outlet opening (31, 31.1) of the building (19), through working areas which are arranged in the direction towards the air flow, to the discharge air openings, wherein a working area in which it is intended that a constant air pressure and cleanliness should prevail is located directly in the region of an air outlet pipe (7).

9. Method according to claim 8, wherein the building (19) which is to be air-conditioned comprises at least two production levels (20, 21), and the air flow introduced by the operation of the ventilation system (22) flows through the building (19) at least partially in a vertical direction.

10. Method according to claim 9, wherein the air flow movement in the building (19) is supported or actually made possible, at least in its vertically flowing sections, by one or more production machines (25) emitting process heat.

11. Method according to any one of claims 8 to 10, wherein the delivery air and the aerosol are delivered in a production level (20) in several working areas which are separated from one another by walls, wherein an introduction of the delivery air and/or of the aerosol is adjusted in individual working areas independently of the introduction of delivery air and/or aerosol in other working areas.

12. Method according to any one of claims 8 to 10, wherein the aerosol to be delivered is enriched with a biologically active substance.

## Revendications

1. Site de production comprenant
- un bâtiment (19) fermé, dans lequel sont disposées une ou de préférence plusieurs zones de travail,
- une installation de ventilation (22)
a) avec au moins un groupe de circulation destiné à acheminer de l'air frais au moyen d'un flux d'air entrant à l'intérieur du bâtiment (19), lequel au moins un groupe de circulation (23 - 23.3) achemine, par une conduite d'air entrant (6), un flux d'air entrant vers au moins un conduit de sortie d'air (7) conformé en tant que conduit textile, disposé dans le bâtiment (19), raccordé au groupe de circulation (23 - 23.3), et
b) avec une ouverture d'air vicié sortant (31, 31.1) réglable par laquelle l'air vicié peut sortir du bâtiment (19),
c) l'au moins un conduit de sortie d'air (7) étant disposé à distance par rapport à l'au moins une ouverture d'air vicié sortant (31, 31.1) de telle sorte que, lors du fonctionnement de l'installation de ventilation (22), une circulation d'air s'instaure dans le bâtiment (19) depuis l'au moins un conduit de sortie d'air (7) vers l'au moins une ouverture d'air vicié sortant (31, 31.1)
- un humidificateur affecté à l'installation de ventilation (22) destiné à acheminer de l'humidité d'air dans le bâtiment (19), lequel humidificateur comprend en tant que générateur de brume au moins un brumisateur à ultrasons disposé dans le groupe de circulation (23 -23.1) afin de générer un aérosol à partir d'un liquide, avec des gouttelettes dont la taille leur permet d'être entraînées par la circulation d'air qui s'instaure dans le bâtiment (19) et au moins un conduit de sortie d'aérosol (14) qui s'étend parallèlement au conduit de sortie d'air (7), installé à faible distance au-dessus de ce dernier, afin que l'aérosol sortant soit capté par la circulation d'air,
- une conduite d'arrivée d'aérosol (6) par laquelle l'aérosol est acheminé vers l'au moins un conduit de sortie d'aérosol (13) et
- des zones de travail disposées dans le sens de la circulation d'air vers la ou les ouvertures d'air vicié sortant, une zone de travail, dans laquelle doit être instaurée une pression d'air constante et qui doit rester propre, se trouvant directement dans la zone du conduit de sortie d'air (7).

2. Site de production selon la revendication 1, dans lequel le bâtiment (19) comporte plusieurs étages parce qu'au moins deux niveaux de production (20, 21) sont prévus, les étages (20, 21) étant raccordés entre eux par au moins un passage de circulation (27) destiné à laisser passer la circulation d'air générée par l'installation de ventilation (22), une ou plusieurs zones de travail étant prévues au niveau de production (20) inférieur et une ou plusieurs zones de travail supplémentaires étant prévues au niveau de production situé au-dessus et l'au moins un conduit de sortie d'air (7) étant disposé au niveau de production (20) inférieur.

3. Site de production selon la revendication 1, dans lequel l'au moins un niveau de production (20) où sont disposés des conduits de sortie d'air (7), est compartimenté en zones de travail délimitées par des murs (32) et dans lequel, au moins deux zones de travail comportent respectivement au moins un conduit de sortie d'air (7) indépendant du ou des conduits de sortie d'air (7) disposés dans les autres zones de travail, dans lequel peut être acheminé une circulation d'air entrant auquel est affecté un conduit de sortie d'aérosol (13).

4. Site de production selon la revendication 2 ou 3, dans lequel les niveaux de production (20, 21) sont raccordés entre eux par au moins un passage de circulation (27) entre les étages afin de laisser passer la circulation d'air générée par l'installation de ventilation (22).

5. Site de production selon l'une des revendications 2 à 4, dans lequel le niveau de production (20) où sont disposés les conduits de sortie d'air (7), est compartimenté en zones de travail délimitées par des murs (32), lesquelles sont raccordées les unes aux autres par au moins un passage de circulation afin de laisser passer la circulation d'air généré par l'installation de ventilation.

6. Site de production selon l'une des revendication 2 à 5, dans lequel est disposé, dans au moins une zone de travail, une machine de production dégageant de la chaleur issue du processus, par exemple un four (25) et que cette zone de travail se trouve à un niveau de production (21) situé au-dessus du niveau de production (20) où sont disposés les conduits de sortie d'air (7).

7. Site de production selon la revendication 6, dans lequel la machine de production (25) dégageant de la chaleur issue du processus est positionnée de telle manière dans le niveau de production (21) que la circulation d'air généré par l'installation de ventilation (22) passe au moins partiellement à côté de celle-ci.

8. Procédé de climatisation d'un bâtiment (19) fermé d'un site de production (18), dans lequel un flux d'air entrant est introduit dans le bâtiment (19) par au moins un conduit de sortie d'air (7) à une pression supérieure à celle existant à cet endroit sans flux d'air entrant, lequel flux d'air entrant est dirigé à travers le bâtiment (19), ou une partie de celui-ci, vers au moins une ouverture de sortie d'air vicié (31, 31.1), dans lequel conduit de sortie d'air (7) est ajouté un aérosol, avec des gouttelettes de liquide dont la taille leur permet d'être entraînées dans le flux d'air qui s'instaure dans le bâtiment (19), l'aérosol étant acheminé dans le conduit de sortie d'aérosol (14) par un conduit d'arrivée d'aérosol (13) en provenance d'un brumisateur à ultrasons disposé dans une installation de ventilation, lequel conduit de sortie d'aérosol (14) s'étend parallèlement au conduit de sortie d'air (7) et est installé à faible distance au-dessus de ce dernier, le flux d'air chargé d'aérosol étant guidé, sur son chemin vers au moins une ouverture de sortie d'air vicié (31, 31.1) du bâtiment (19), à travers les zones de travail qui sont disposées dans le sens de la circulation d'air vers la ou les ouvertures d'air vicié, une zone de travail dans laquelle doit s'instaurer une pression d'air constante et qui doit rester propre, se trouvant directement dans la zone d'un conduit de sortie d'air (7).

9. Procédé selon la revendication 8, dans lequel le bâtiment (19) à climatiser présente au moins deux niveaux de production (20, 21) et la circulation d'air s'instaurant lors de la mise en service de l'installation de ventilation (22) traversant au moins partiellement le bâtiment (19) dans le sens vertical.

10. Procédé selon la revendication 9, dans lequel le mouvement du flux d'air dans le bâtiment (19) est soutenu ou devient possible au moins dans les tronçons de circulation verticale grâce à une ou plusieurs machines de production (25) dégageant de la chaleur issue du processus.

11. Procédé selon l'une des revendications 8 à 10 dans lequel l'air entrant et l'aérosol sont guidés à un niveau de production (20) dans différentes zones de travail séparées les unes des autres par des murs, un acheminement de l'air entrant et/ou de l'aérosol étant mis à l'arrêt dans certaines zones de travail indépendamment de l'acheminement d'air entrant et/ou d'aérosol dans les autres zones de travail.

12. Procédé selon l'une des revendications 8 à 10 dans lequel l'aérosol à acheminer est enrichi avec un agent actif biologique.
